# EUROPÄISCHE PATENTSCHRIFT

(11) **EP 2 087 922 B1**
(45) Veröffentlichungstag und Bekanntmachung des Hinweises auf die Patenterteilung: **09.06.2010**
(21) Anmeldenummer: 09150366.4
(22) Anmeldetag: 12.01.2009
(51) Int. Cl.: A61N 1/05, A61M 25/09, A61N 1/372

(54) **Multipolarer Führungsdraht und Elektrodenleitung**
Multipolar lead wire and electrode lead
Fil-guide multipolaire et sonde d'électrodes

(30) Priorität: 05.02.2008 DE 102008007542
(43) Veröffentlichungstag der Anmeldung: 12.08.2009
(73) Patentinhaber: Biotronik CRM Patent AG, 6341 Baar (CH)
(72) Erfinder: Dörr, Thomas, 12437 Berlin (DE)
(74) Vertreter: Lindner-Vogt, Karin L.

(56) Entgegenhaltungen:
- EP-A- 1 062 969
- EP-A- 1 062 970
- WO-A-95/09561
- WO-A-2007/075974

## Beschreibung

Die Erfindung betrifft eine multipolare Elektrodenanordnung für ein Elektrostimulationsgerät wie beispielsweise einen implantierbaren Herzschrittmacher, Kardioverter/Defibrillator oder dergleichen.

Eine solche Elektrodenanordnung besitzt an ihrem proximalen Ende einen elektrischen Anschluss mit elektrischen Kontakten für die Kontaktierung entsprechender Gegenkontakte eines Anschlusses des Elektrostimulationsgerätes. Am distalen Ende finden sich mehrere elektrisch leitende Oberflächenbereiche, auch als Pole, Elektroden z. B. Ring- oder Tip-Elektroden) oder Elektrodenpole bezeichnet, die elektrisch über entsprechende Zuleitungen mit den elektrischen Kontakten des Anschlusses der Elektrodenleitung verbunden sind.

Multipolare Elektrodenleitungen, die z. B. zur Neurostimulation eingesetzt werden, besitzen einen entsprechend komplexen elektrischen Anschluss in Form eines Elektrodensteckers mit z. B. 8 Kontakten. Des Weiteren wird die Anzahl der realisierbaren Elektrodenpole durch die Zahl der möglichen Elektrodenzuleitungen begrenzt.

Für die Anwendung der kardialen Stimulation (z. B. Coronar Sinus Elektroden für die Resynchronisationstherapie) haben sich bislang die multipolaren Elektroden nicht durchgesetzt, da die derzeit gültigen Steckernormen (IS-1, zukünftig IS-4) den Einsatz multipolarer Elektroden nicht unterstützen.

Für die endgültige Anwendung werden bei multipolaren Elektrodenleitungen jedoch nur einige der vorhandenen Elektrodenpole genutzt. Deren Auswahl geschieht entweder durch elektrische Prüfung nach Implantation oder bereits vor der Implantation anhand der anatomischen Gegebenheiten.

In US 6,859,667 wird die Integration eines Multiplexers in einer Elektrode mit dem Ziel, zu bestehenden Steckernormen kompatibel zu bleiben, beschrieben. Hier wird jedoch eine dauerhafte aktive Ansteuerung des Multiplexers durch das elektronische Implantat vorausgesetzt. Nachteil dieser vorbekannten Lösung besteht darin, dass immer ein an die Elektrode und dessen Multiplexer angepasstes Implantat vorausgesetzt wird.

In US 6,418,348 wird ebenso ein in das distale Ende der Elektrode integrierter Multiplexer beschrieben. Auch hier ist vorausgesetzt, dass das elektronische Implantat die Elektrodenkonfiguration durchführt und damit eine entsprechende Steckerkonfiguration und Gerätekonfiguration nötig ist. Der Anschluss dieser Elektrode ist nicht an herkömmliche elektronische Implantate möglich.

Aus EP 1 062 970 ist eine Elektrodenleitung bekannt, die zwischen einer zentralen Zuleitung und einem jeweiligen Elektrodenpol elektronische oder mechanische Schaltmittel aufweist, mit denen ein Elektrodenpol dauerhaft mit der zentralen Zuleitung verbunden werden können oder dauerhaft von der zentralen Zuleitung getrennt werden können, so dass im Ergebnis nur einige ausgewählte Elektrodenpole über die Zuleitung mit den elektrischen Kontakten des Anschlusses der Elektrodenleitung für die Abgabe von Stimulationsimpulsen oder die Aufnahme elektrische Potentiale verbunden sind.

Die US 4,628.934 zeigt eine Elektrodenleitung, in deren elektrischem Anschluss (z. B. dem Elektrodenstecker) oder in deren Elektrodenzuleitung (vorzugsweise am distalen Ende der Elektrodenleitung) elektrisch einmalig programmierbare Schaltmittel integriert sind, die mittels eines elektronischen Konfigurationsprozesses durch ein externes Gerät vor oder nach Implantation derart konfiguriert werden können, dass die Kontakte des elektrischen Anschlusses der Elektrodenleitung anschließend jeweils einem oder mehreren der Elektrodenpole der Elektrodenleitung zugeordnet sind. Durch die elektrisch einmalig programmierbaren Schaltmittel ist somit bei Abschluss des Konfigurationsprozesses dauerhaft und ohne dass es hierzu weiterer Energie bedarf, festgelegt, welche Elektrodenpole mit welchem Kontakt des elektrischen Anschlusses der Elektrodenleitung elektrisch leitend verbunden sind und welche nicht.
Nachteilig an dieser Lösung ist, dass das Implantat nicht standardmäßig aufgebaut werden kann, weil zusätzliche Ansteuerungsmittel vorzusehen sind, die für die Programmierung der Leitungen notwendig sind.

Daher besteht die Aufgabe, eine "programmierbare" Elektrodenanordnung so weiterzuentwickeln, dass sie einfach und leicht mit Standard-Implantaten verwendet werden kann. Die Aufgabe wird gemäß Anspruch 1 so gelöst, dass die Elektrodenleitung ein Lumen zum Einführen eines Führungsdrahtes aufweist, das mit elektrischen Kontakten versehen ist, die mit den elektrischen Schaltmitteln verbunden sind, so dass die elektrischen Schaltmittel über einen mit elektrischen Gegenkontakten versehenen Führungsdraht anzusteuern sind, wenn ein solcher Führungsdraht in das Lumen eingeführt ist.

So ist die für die "Programmierung" benötigte elektrische Verbindung vor dem Anschluss der Elektrodenleitung an ein elektronisches Implantat vollständig entfernbar und es müssen beispielsweise keine zusätzlichen Steuerungsleitungen vorgesehen werden, über die gemäß des Standes der Technik die "Programmierung" erfolgt. Vorzugsweise ist für die für die "Programmierung" benötigte elektrische Verbindung zwischen Multiplexer, den elektrisch dauerhaft konfigurierbaren Schaltmitteln und dem Programmier- und Testgerät ein dafür ausgeprägter Führungsdraht (Mandrin) vorgesehen. Hierzu weist die Elektrodenleitung vorzugsweise ein Lumen zum Einführen eines solchen Führungsdrahtes auf, das mit elektrischen Kontakten versehen ist, die mit dem Multiplexer verbunden sind, so dass der Multiplexer über einen mit elektrischen Gegenkontakten versehenen Mandrin anzusteuern ist, wenn ein solcher Mandrin in das Lumen eingeführt ist.

Die Konfiguration erfolgt dabei mittels nicht-reversibler elektronischer oder elektrischer Bauelemente als Schaltmittel, wie z. B. durch Sicherungen (Fuses) oder durch elektronische Bauelemente wie Zener-Dioden (Zener-Zapping), wobei durch die einmalige Programmierung entweder eine vorhandene elektrische Verbindung dauerhaft unterbrochen wird (Sicherung durchbrennen) oder eine gesperrte Halbleiterverbindung dauerhaft leitfähig gemach wird (z. B. durch Zener-Zapping).

Vorteile dieser Lösung sind zum einen die Möglichkeit, eine multipolare Elektrode mit den derzeit gültigen Steckernormen (z. B. IS-1, zukünftig IS-4) verknüpfen zu können. Dabei sind vor allem die Vorteile bezüglich der Kompatibilität zwischen Elektroden und Geräten verschiedener Hersteller als auch die Aspekte der Zuverlässigkeit (weniger Kontaktstellen) hervorzuheben.

Des Weiteren gestattet diese Technologie bei Integration in die Elektrodenspitze die Entwicklung von multipolaren Elektroden mit wesentlich mehr Elektrodenpolen bei gleichbleibender oder nur geringfügiger Zunahme der Anzahl von Elektrodenleitungen und somit bei unverändertem Durchmesser der Elektrode gegenüber herkömmlichen multipolaren Elektroden.

Gemäß einer bevorzugten Ausführungsvariante ist in die Elektrodenleitung ein Multiplexer derart integriert, dass die Elektrodenpole vor der dauerhaften Konfiguration wahlweise mit einem jeweiligen Kontakt des Anschlusses zu verbinden sind, um zunächst verschiedene Elektrodenkonfigurationen testen zu können. Der in die Elektrodenleitung integrierte Multiplexer dient somit der Bestimmung einer geeigneten Elektrodenkombination und erlaubt es vor der dauerhaften Konfiguration (Programmierung) der Elektrodeleitung die möglichen Elektrodenkonfigurationen temporär einzustellen.

Die temporäre Konfiguration der Elektrode kann dabei vorzugsweise über ein externes Programmier- und Testgerät" erfolgen. Dieses "Programmier- und Testgerät" beinhaltet alle Funktionen zum Testen einer jeweiligen Elektrodenkonfiguration sowie die Ansteuerung des Multiplexers für das temporäre Testen und die Programmiereinrichtung für die dauerhafte Konfiguration (Programmierung) der Elektrodenleitung.

Eine weitere Aufgabe besteht darin, ohne aufwendige Zusatzfunktionen im Implantat oder in der Elektrodenanordnung selbst die beschriebene "Programmierung" der Elektrodenanordnung vornehmen zu können.

Dazu wird der Führungsdraht aus dem Stand der Technik genutzt. Dieser Führungsdraht beispielsweise aus der WO 95/09561 umfasst einen lang gestreckten Körper, der ein proximalen Ende, welches sich nach Einbringung des Führungsdrahtes außerhalb eines menschlichen oder tierischen Körpers befindet, und ein distalen Ende, welches in einen Körper oder in das Lumen einer Elektrodenleitung eingeführt werden kann, umfasst. Dieser dient dazu, Elektrodenanordnungen sicher an den Behandlungsort zu führen. Unter Führungsdraht im Sinne der Anmeldung wird auch ein Mandrin verstanden, welcher in einer Elektrodenanordnung eingeführt ist, um beispielsweise eine aktive Fixierung zu betätigen.

Weiterhin weist dieser Führungsdraht am distalen Ende mindestens einen elektrisch leitenden Kontakt auf, welcher über mindestens eine Leitung elektrisch leitend mit dem proximalen Ende verbunden ist und welcher ausgebildet ist, die elektrischen Schaltmittel der Elektrodenleitung Anspruch 1 zu kontaktieren.

So wird die Programmierung erleichtert und die Elektrodenleitung kann mit herkömmlichen Implantaten genutzt werden, die keine zusätzlichen Schalt- oder Programmiermittel aufweisen müssen. Auch kann dadurch der Aufbau der Elektrodenleitung einfach gehalten werden, was zu einer Durchmesserverringerung aufgrund weniger elektrischer Leitungen führt.

Der Führungsdraht kann am proximalen Ende einen elektrischer Anschluss aufweisen, der über die elektrische Leitung mit den Kontakten verbunden ist und welcher ausgebildet ist, mit einem externen Test- und Programmiergerät verbunden zu werden. So wird die "Programmierung" der Elektrodenleitung nach Anspruch 1 für den Anwender weiter vereinfacht, da durch diesen, vorteilhafterweise genormten Anschluss, eine Fehlprogrammierung verhindert wird.

Auch kann die mindestens eine elektrische Leitung im oder am Führungsdraht isoliert geführt werden..

Die Erfindung wird nun Anhand von Ausführungsbeispielen mit Bezug auf die Figuren näher erläutert. Diese zeigen in:
- Fig. 1:: ein distales Ende einer multipolaren Elektrodenleitung;
- Fig. 2a:: ein Blockschaltbild einer ersten Variante einer multipolaren Elektrodenlei- tung;
- Fig. 2b:: ein Blockschaltbild einer zweiten Variante einer multipolaren Elektrodenlei- tung;
- Fig. 3:: eine Schaltung zur dauerhaften Konfiguration eines einzelnen Elektroden- pols für eine Elektrodenleitung gem. Fig. 2a oder Fig. 2b;
- Fig. 4:: eine Elektrodenleitung mit einem in einen Elektrodenleitungsstecker integ- rierten Multichipmodul; und
- Fig. 5:: eine Elektrodenleitung mit mehreren, im Bereich von deren distalem Ende angeordneten Multichipmodulen.

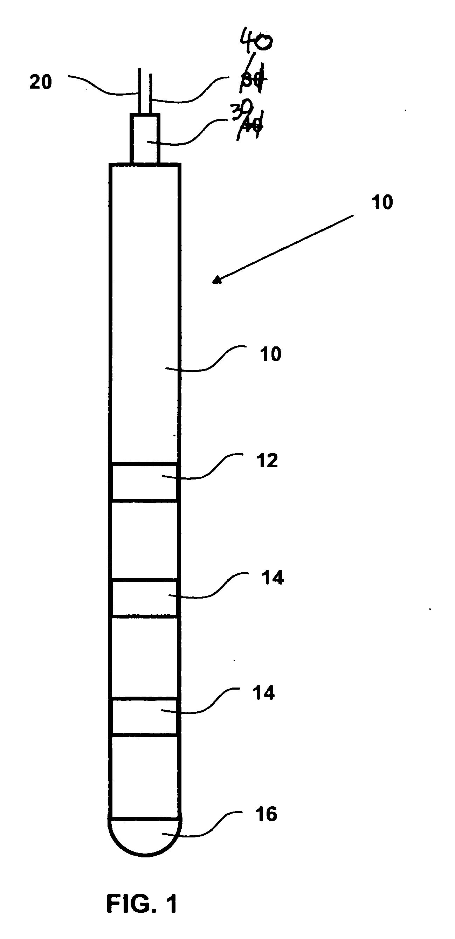

Der schematischen Darstellung eines distalen Endes einer multipolaren Elektrodenleitung 10 in Fig. 1 ist zu entnehmen, dass die multipolare Elektrodenleitung eine Elektrodenleitung 12 im engeren Sinne aufweist, die mehrere elektrisch leitende Oberflächenabschnitte als Elektrodenpole aufweist, nämlich zum einen Ringelektroden 14 und zum anderen eine Chipelektrode oder Spitzenelektrode 16. Um die Elektrodenpole 14 und 16 mit elektrischen Kontakten eines in Fig. 1 nicht dargestellten elektrischen Anschlusses am proximalen Ende der Elektrodenleitung 10 elektrisch zu verbinden, sind im Inneren der Elektrodenleitung 12 elektrische Zuleitungen 20, 30 und 40 vorgesehen, von denen die Zuleitungen 20 und 40 die Form eines einfachen Drahtes haben, während die elektrische Zuleitung 30 die Form einer Drahtwendel hat, die im Inneren ein Lumen einschließt. Die elektrischen Zuleitungen 20 und 40 können außerhalb oder innerhalb des Lumens der die elektrische Zuleitung 30 bildenden Drahtwendel angeordnet sein.

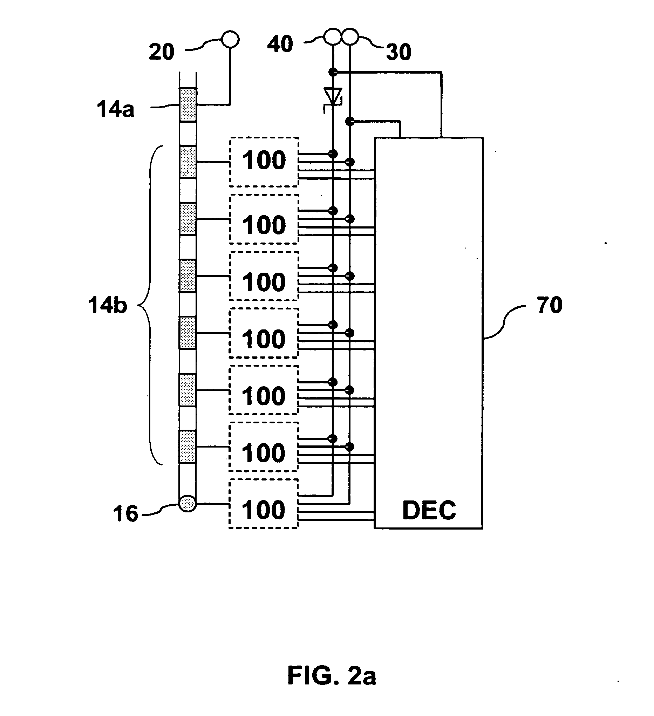

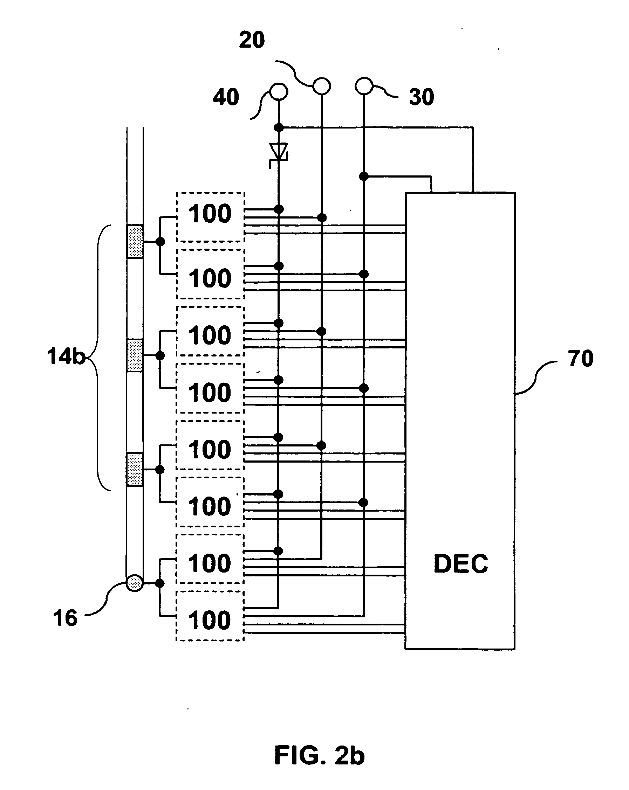

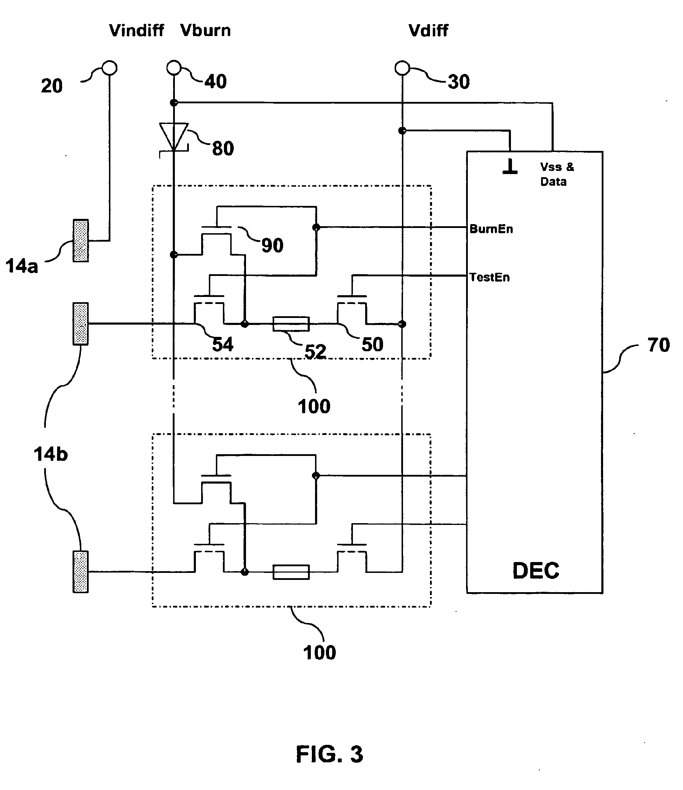

Zwischen wenigstens einigen der Elektrodenpole 14 und 16 und wenigstens einigen der elektrischen Zuleitungen 20, 30 oder 40 ist erfindungsgemäß eine Schaltung angeordnet, um einen jeweiligen Elektrodenpol wahlweise dauerhaft mit einer der elektrischen Zuleitungen elektrisch zu verbinden oder von dieser zu trennen. Dies wird mit Bezug auf die Figuren 2a, 2b und 3 nachfolgend näher erläutert.

In Abbildung 2a ist das Blockschaltbild einer multipolaren Elektrode mit einem festgelegtem Elektrodenpol 14a, der mit einem elektrischen Kontakt eines elektrischen Anschlusses der Elektrodenleitung über die Zuleitung 20 fest elektrisch verbunden ist, und mit sieben programmierbaren Elektrodenpolen 14b, 16, die jeweils über eine als "Programmiereinheit" dienende elektrische Schaltung 100, wie sie nachfolgend mit Bezug auf Figur 3 näher erläutert ist, mit einer zweiten Zuleitung 30 verbunden sind. Die elektrische Schaltung 100 ist wiederum mit einem Multiplexer 70, der gemeinsamen Elektrodenzuleitung 30 und einer als "Programmierleitung" dienenden elektrischen Zuleitung 40 verbunden. Eine solche multipolare Elektrodenleitung 10 ist z. B. als Coronar-Sinus-Elektrodenleitung mit elektronisch "verschiebbarer" Ringelektrode für die kardiale Resynchronisationstherapie geeignet.

Mit Hilfe des Multiplexers 70 kann nämlich eine jeweilige elektrische Schaltung 100 so angesteuert werden, dass diese den jeweils zugeordneten Elektrodenpol 14b oder 16 mit elektrischen Zuleitungen 30 verbindet oder diesen von ihr trennt. Wie nachfolgend in Bezug auf Fig. 3 näher erläutert, kann dies zunächst vorläufig geschehen, um testweise jeweils einen beispielsweise der Elektrodenpole 14b mit einem Kontakt des Anschlusses am proximalen Ende der Elektrodenleitung zu verbinden. Indem immer wieder ein anderer der Elektrodenpole 14b mit der Zuleitung 30 verbunden wird, ergibt sich quasi eine wandernde Ringelektrode. Anschließend kann die elektrische Verbindung zwischen einem jeweiligen Elektrodenpol 14b und der Zuleitung 30 dauerhaft hergestellt oder dauerhaft unterbunden werden.

Abbildung 2b zeigt das Blockschaltbild einer 4-fach multipolaren Elektrodenleitung, bei der kein Pol fest mit einem Kontakt des elektrischen Anschlusses der Elektrodenleitung verbunden ist, Vielmehr kann jeder Elektrodenpol 14b oder 16 wahlweise dauerhaft über die Zuleitung 30 der die Zuleitung 40 mit dem einen oder dem anderen Kontakt beispielsweise eines Elektrodenleitungssteckers gemäß IS-1 Standard elektrisch verbunden werden. Hierfür ist die in Figur 3 dargestellte "Programmiereinheit" 100 jeweils 2mal pro Elektrodenpol 14b oder 16 vorhanden und dabei jeweils mit einer der zwei gemeinsamen Elektrodenzuleitungen 20 oder 30 und der "Programmierleitung" 40 verbunden. Eine solche Anordnung könnte z. B. in der Neurostimulation für die Rückenmarkstimulation (Spinal Cord Stimulation) eingesetzt werden.

Eine beliebige Vervielfachung der Multiplexerstruktur gestattet den Aufbau von Elektroden mit mehr als 2 gemeinsamen Elektrodenleitungen, wie z. B. eine Elektrode mit 8 Steckerkontakten und z. B. 16 oder 32 frei konfigurierbaren Elektrodenpolen.

Figur 3 zeigt eine mögliche Schaltung zur dauerhaften Konfiguration eines einzelnen Elektrodenpols. Eine gemeinsame Zuleitung 30 ist zunächst mit mehreren Elektrodenpolen verbunden ist. Diese Zuleitung 30 ist jeweils über einen ersten MOS-Transistor [Verarmungstyp] 50. eine Sicherung 52 und einen zweiten MOS-Transistor [Verarmungstyp] 54 mit dem auszuwählenden Elektrodenpol 14b verbunden. Aufgrund des eingesetzten Verarmungstyps der MOS-Transistoren sind zunächst so alle Elektrodenpole 14b mit der gemeinsamen Zuleitung 30 verbunden.

Zur dauerhaften "Programmierung" eines Elektrodenpols wird über die zusätzliche Elektrodenzuleitung 40 ein Strom eingespeist, der die Sicherung 52 durchbrennt, so dass der so programmierte Elektrodenpol dauerhaft von der gemeinsamen Leitung 30 getrennt wird. Die Auswahl des zu "programmierenden" Elektrodenpols geschieht dabei über einen Multiplexer 70 als Decoder, der seine Spannungsversorgung über die zusätzliche Elektrodenzuleitung 40, abgegriffen vor einer Z-Diode 80 und die gemeinsame Elektrodenzuleitung 30 erhält. Die Ansteuerung des Multiplexers 70 erfolgt über ein auf die Spannungsversorgung aufmoduliertes Signal, so dass zusätzliche Steuerleitungen entfallen können. Für die dauerhafte Programmierung wird ein jeweiliger zu programmierender Elektrodenpol 14b vom Multiplexer 70 mittels des Signals BurnEn ausgewählt. Dieses Signal öffnet einen Transistor 90 und sperrt den Transistor 54 um eine Stromabgabe in das Gewebe während des "Programmiervorganges" zu verhindern. Gleichzeitig wird die Spannung der Zuleitung 30 derart erhöht, dass die Schwellspannung der Z-Diode 80 überschritten wird und die Sicherung 52 durchgebrannt wird. Mit diesem Vorgang ist die dauerhafte Abschaltung (Programmierung) eines oder mehrerer Elektrodenpole abgeschlossen.

Für die temporäre Testung stellt der Multiplexer 70 den Ausgang TestEn zur Verfügung. Mittels dieses Signals können für temporäre Testungen ein oder mehrere Elektrodenpole von der gemeinsamen Elektrodenleitung 30 getrennt werden, indem der MOS-Verarmungstransistor 50 gesperrt wird.

Zur vollständigen Übersicht sind in diesem Realisierungsbeispiel noch der differente Elektrodenpol 14a und dessen Zuleitung 20 dargestellt, der in diesem Beispiel immer fest verdrahtet ist.

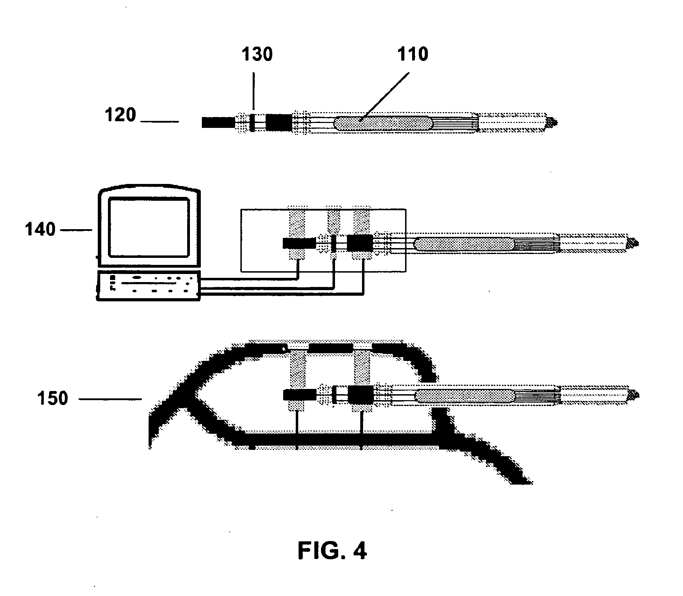

In Figur 4 ist die Integration eines Multichipmodules 110 zur dauerhaften Elektrodenprogrammierung in einen bipolaren Anschluss 120 am proximalen Ende der Elektrodenleitung 10 gemäß IS-1-Standard dargestellt. Der Kontakt 130 der Zuleitung 40 für die Programmierung ist dabei derart angeordnet, dass dieser ausschließlich von einem externen Test- und Programmiergerät 140 kontaktiert wird und bei Verbindung der Elektrodeleitung 10 mit einem elektronischen Implantat 150 isoliert bleibt.

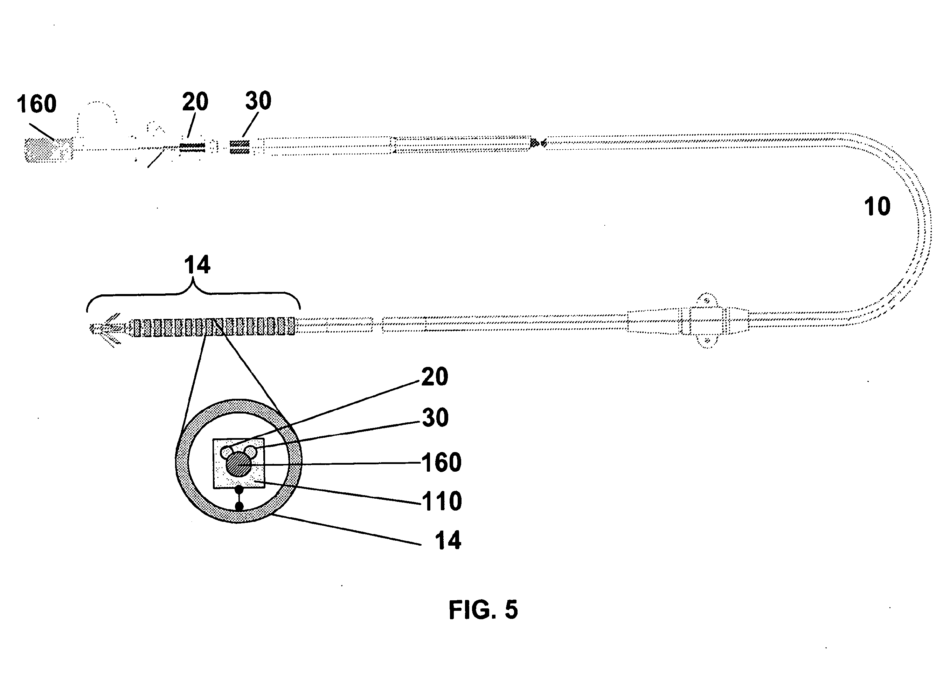

Figur 5 zeigt eine Elektrodenleitung 10 mit mehreren Multichipmodulen 110, die im Bereich der Elektrodenpole 14 in die Elektrodenleitung 10 integriert sind. Die elektrische Verbindung dieser Multichipmodule 110 mit einem externen Test- und Programmiergerät 140 erfolgt in diesem Fall mit einem speziell dafür ausgeprägten Führungsdraht 160 der zur Konfiguration der Elektrodenleitung 10 mit seinem proximalen Ende elektrisch mit dem externen Test- und Programmiergerät 140 verbunden ist und mit entsprechenden elektrischen Kontakten an seinem distalen Ende die Multichipmodule 110 kontaktiert. Dieser Führungsdraht wird vor dem Anschluss der Elektrodenleitung 10 an das elektronische Implantat 150 entfernt. Die Elektrodenleitung 10 weist hierzu ein Lumen auf, in das der Führungsdraht 160 einzuführen ist und welches im Bereich der Multichipmodule 110 elektrische Kontakte aufweist, die elektrisch mit den Multichipmodulen 110 verbunden sind.

Im Querschnitt ist ferner die dauerhafte Verbindung des Multichipmoduls eines Elektrodenpols mit den beiden Elektrodenzuleitungen 20 und 30 dargestellt.

## Patentansprüche

1. Elektrodenanordnung für ein Elektrostimulationsgerät, die eine Elektrodenleitung (10) mit einem elektrischen Anschluss (120) am proximalen Ende der Elektrodenleitung und mit mehreren leitenden Oberflächenbereichen (14, 16) im Bereich des distalen Endes der Elektrodenleitung zur Abgabe elektrischer Impulse an ein Herz oder zur Aufnahme elektrischer Signale von einem Herzen aufweist, wobei
- die elektrisch leitenden Oberflächenbereiche wenigstens teilweise mit dem elektrischen Anschluss am proximalen Ende der Elektrodenleitung elektrisch verbunden sind, und
- wobei die Elektrodenanordnung in dem elektrischen Anschluss (120) oder in der Elektrodenleitung (12) angeordnete elektrische Schaltmittel (100) aufweist, die elektrisch zwischen wenigstens einem Anschlusskontakt des elektrischen Anschlusses und wenigstens jeweils einigen der elektrisch leitenden Oberflächenbereiche (14b, 16) angeordnet und ein zunächst elektrisch leitendes oder nicht-leitendes Bauelement (52) aufweisen, das durch einen dauerhaften Konfigurationsprozess durch einen elektrischen Schaltimpuls dauerhaft elektrisch nicht-leitend bzw. leitend zu machen ist, so dass die jeweils zugeordnete elektrisch leitende Oberfläche (14b, 16) anschließend dauerhaft mit einem Anschlusskontakt des elektrischen Anschlusses verbunden ist bzw. dauerhaft nicht mehr elektrisch mit einem Anschlusskontakt des elektrischen Anschlusses verbunden ist.
**dadurch gekennzeichnet, dass**
die Elektrodenleitung ein Lumen zum Einführen eines Führungsdrahtes (160) aufweist, das mit elektrischen Kontakten versehen ist, die mit den elektrischen Schaltmitteln (100) verbunden sind, so dass die elektrischen Schaltmittel (100) über einen mit elektrischen Gegenkontakten versehenen Führungsdraht (160) anzusteuern sind, wenn ein solcher Führungsdraht (160) in das Lumen eingeführt ist.

2. Elektrodenanordnung nach Anspruch 1, **dadurch gekennzeichnet, dass** das zunächst elektrisch nicht-leitende Bauelement jeweils eine Zener-Diode ist.

3. Elektrodenanordnung nach Anspruch 1 oder 2, **dadurch gekennzeichnet, dass** in die Elektrodenleitung (10) ein Multiplexer (70) derart integriert ist, dass wählbare elektrisch leitende Oberflächen vor dem dauerhaften Konfigurationsprozess wahlweise mit einem jeweiligen Kontakt des elektrischen Anschlusses der Elektrodenleitung zu verbinden sind.

4. Elektrodenanordnung nach einem der Ansprüche 1 bis 3, **dadurch gekennzeichnet, dass** die Elektrodenleitung (10) eine als Programmierleitung dienende Zuleitung (40) aufweist, die zum Zweck der Ansteuerung der elektrischen Schaltmittel (100) mit diesen verbunden ist verbunden ist.

5. Elektrodenanordnung nach Anspruch 3 und 4, **dadurch gekennzeichnet, dass** die als Programmierleitung dienende Zuleitung (40) mit dem Multiplexer (70) verbunden ist.

6. Elektrodenanordnung nach einem der Ansprüche 1 bis 5, **dadurch gekennzeichnet, dass** der elektrische Anschluss (120) am proximalen Ende der Elektrodenleitung (10) weitestgehend dem IS-1 Standard entspricht und einen zusätzlichen, dritten Kontakt aufweist, der mit einer als Programmierleitung dienenden Zuleitung (40) der Elektrodenleitung (10) verbunden ist.

7. Elektrodenanordnung nach einem der Ansprüche 1 bis 6, **dadurch gekennzeichnet, dass** der Führungsdraht (160) zur Führung und Programmierung der Elektrodenleitung (10) einen lang gestreckten Körper mit einem proximalen Ende, welches sich nach Einbringung des Führungsdrahtes außerhalb eines menschlichen oder tierischen Körpers befindet, und einem distalen Ende, welches in einen Körper oder in das Lumen einer Elektrodenleitung eingeführt werden kann, umfasst, und an seinem distalen Ende mindestens einen elektrisch leitenden Kontakt aufweist, welcher über mindestens eine Leitung elektrisch leitend mit dem proximalen Ende verbunden ist und welcher ausgebildet ist, die elektrischen Schaltmittel (100) der Elektrodenleitung zu kontaktieren.

8. Elektrodenanordnung nach Anspruch 7, **dadurch gekennzeichnet, dass** der Führungsdraht (160) am proximalen Ende einen elektrischen Anschluss aufweist, der über die elektrische Leitung mit den Kontakten verbunden ist und welcher ausgebildet ist, mit einem externen Test- und Programmiergerät (140) verbunden zu werden.

9. Elektrodenanordnung nach Anspruch 7 oder 8, **dadurch gekennzeichnet, dass** die elektrische Leitung isoliert geführt ist.

## Claims

1. An electrode arrangement for an electrostimulation device having an electrode line (10) with an electric terminal (120) on the proximal end of the electrode line and having a plurality of conducting surface areas (14, 16) in the area of the distal end of the electrode line for delivering electric pulses to the heart or for receiving electric signals from the heart, wherein
- the electrically conducting surface areas are at least partially electrically connected to the electric terminal on the proximal end of the electrode line, and
- wherein the electrode arrangement has electric switching means (100) arranged in the electric terminal (120) or in the electrode line (12), these means being arranged electrically between at least one terminal contact of the electric terminal and at least a few of the electrically conducting surface areas (14b, 16) and having a component (52), which is initially electrically conductive or nonconductive and may be rendered permanently nonconductive and/or conductive, respectively, by a permanent configuration process by an electric switching pulse, so that the respective particular electrically conductive surface (14b, 16) is then connected permanently to a terminal contact of the electric terminal and/or is permanently no longer connected electrically to the terminal contact of the electric terminal,
**characterized in that**
the electrode line has a lumen for insertion of a guide wire (160), said lumen being provided with electric contacts which are connected to the electric switching means (100), so that the electric switching means (100) are to be triggered via a guide wire (160) provided with electric mating contacts when such a guide wire (160) is inserted into the lumen.

2. The electrode arrangement according to claim 1, **characterized in that** the initially electrically nonconductive component is a zener diode in each case.

3. The electrode arrangement according to claim 1 or 2, **characterized in that** a multiplexer (70) is integrated into the electrode line (10), such that selectable electrically conductive surfaces are optionally connected to a particular contact of the electric terminal of the electrode line prior to the permanent configuration procedure.

4. The electrode arrangement according to claims 1 to 3, **characterized in that** the electrode line (10) has a lead (40), which serves as a programming line which, for the purpose of triggering of the electric switching means (100), is connected thereto.

5. The electrode arrangement according to claim 3 and 4, **characterized in that** the lead (40), which serves as the programming line, is connected to the multiplexer (70).

6. The electrode arrangement according to any one of claims 1 to 5, **characterized in that** the electric terminal (120) on the proximal end of the electrode line (10) corresponds largely to the IS-1 standard and has an additional third contact, which is connected to a lead (40) of the electrode line (10) serving as the programming line.

7. The electrode arrangement according to any one of claims 1 to 6, **characterized in that** the guide wire (160) for guiding and programming the electrode line (10) has an elongated body with a proximal end that is located outside of the human or animal body after insertion of the guide wire, and comprises a distal end that is insertable into the body or into the lumen of an electrode line and has on its distal end at least one electrically conductive contact, which is electrically connected to the proximal end by at least one line and is designed to contact the electric switching means (100) of the electrode line.

8. The electrode arrangement according to claim 7, **characterized in that** the guide wire (160) has an electric terminal on the proximal end, which is connected to the contacts via the electric line and which is designed to be connected to an external testing and programming device (140).

9. The electrode arrangement according to claim 7 or 8, **characterized in that** the electric line is carried with insulation.

## Revendications

1. Système d'électrode pour appareil de stimulation à électrode qui comporte un câble d'électrode (10) à raccordement électrique (120) à l'extrémité proximale du câble d'électrode et plusieurs zones surfaciques conductrices (14, 16) au niveau de l'extrémité distale du câble d'électrode pour émettre des impulsions électriques vers un coeur ou pour recevoir des signaux électriques d'un coeur,
- les zones surfaciques conductrices d'électricité étant reliées électriquement au moins partiellement au raccordement électrique à l'extrémité proximale du câble d'électrode, et
- le système d'électrode présentant des moyens de commutation électriques (100) disposés dans le raccordement électrique (120) ou dans le câble d'électrode (12), qui sont disposés au niveau électrique entre au moins un contact de raccordement du raccordement électrique et au moins respectivement certaines des zones surfaciques conductrices d'électricité (14b, 16) et présentent un élément de construction (52) d'abord conducteur ou non conducteur d'électricité qui peut être rendu permanemment non conducteur ou conducteur d'électricité par un processus de configuration permanent via une impulsion de commutation électrique, de sorte que la surface conductrice d'électricité (14b, 16) respectivement associée est ensuite reliée de manière permanente à un contact de raccordement du raccordement électrique ou n'est plus reliée électriquement de manière permanente à un contact de raccordement du raccordement électrique,
**caractérisé en ce que**
le câble d'électrode présente un lumen pour l'introduction d'un fil de guidage (160), lequel lumen est pourvu de contacts électriques qui sont reliés aux moyens de commutation électriques (100), de sorte que les moyens de commutation électriques (100) se commandent par un fil de guidage (160) pourvu de contre-contacts lorsqu'un tel fil de guidage (160) est introduit dans le lumen.

2. Système d'électrode selon la revendication 1, **caractérisé en ce que** l'élément de construction d'abord non conducteur d'électricité est respectivement une diode Zener.

3. Système d'électrode selon la revendication 1 ou 2, **caractérisé en ce que**, dans le câble d'électrode (10), un multiplexeur (70) est intégré de manière à ce que des surfaces conductrices d'électricité sélectionnables se relient avant le processus de configuration permanent au choix à un contact respectif du raccordement électrique du câble d'électrode.

4. Système d'électrode selon une des revendications 1 à 3, **caractérisé en ce que** le câble d'électrode (10) présente une ligne d'alimentation (40) servant de ligne de programmation qui, aux fins de commander les moyens de commutation électriques (100), est reliée à ceux-ci.

5. Système d'électrode selon les revendications 3 et 4, **caractérisé en ce que** la ligne d'alimentation (40) servant de ligne de programmation est reliée au multiplexeur (70).

6. Système d'électrode selon une des revendications 1 à 5, **caractérisé en ce que** le raccordement électrique (120) correspond, à l'extrémité proximale du câble d'électrode (10), dans une large mesure à la norme IS-1 et présente un troisième contact supplémentaire qui est relié à une ligne d'alimentation (40) servant de ligne de programmation du câble d'électrode (10).

7. Système d'électrode selon une des revendications 1 à 6, **caractérisé en ce que** le fil de guidage (160) servant au guidage et à la programmation du câble d'électrode (10) comprend un corps étiré en longueur ayant une extrémité proximale qui, après introduction du fil de guidage, se trouve en dehors d'un organisme humain ou animal et une extrémité distale qui peut être introduite dans un organisme ou dans le lumen d'un câble d'électrode et présente à son extrémité distale au moins un contact conducteur d'électricité qui est relié par au moins un câble avec conduction d'électricité à l'extrémité proximale et qui est réalisé de manière à être en contact avec les moyens de commutation électriques (100) du câble d'électrode.

8. Système d'électrode selon la revendication 7, **caractérisé en ce que** le fil de guidage (160) présente à son extrémité proximale un raccordement électrique qui est relié par la ligne électrique aux contacts et qui est réalisé de manière à être relié à un appareil externe de test et de programmation (140).

9. Système d'électrode selon la revendication 7 ou 8, **caractérisé en ce que** la ligne électrique est guidée sous isolation.
